# EUROPEAN PATENT APPLICATION

(11) **EP 4 810 027 A1**
(43) Date of publication of application: **23.09.2026**
(21) Application number: 25211104.2
(22) Date of filing: 24.10.2025
(51) Int. Cl.: A61K 9/46, A61K 31/165

(54) **AN EFFERVESCENT TABLET FORMULATION OF LISDEXAMFETAMINE DIMESYLATE**

(30) Priority: 20.03.2025 TR 2025003339
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); ATAMAN, Seval, Istanbul (TR); YUCEL, Alpay, Istanbul (TR)
(74) Representative: Genç Ilhan, Oznur

(57) **Abstract**

The present invention relates to an effervescent tablet formulation comprising lisdexamfetamine dimesylate and at least one excipient. Furthermore, the invention discloses a process which is simple, rapid, cost effective, time-saving and industrially convenient process.

## Description

### Field of the Invention

The present invention relates to an effervescent tablet formulation comprising lisdexamfetamine dimesylate and at least one excipient. Furthermore, the invention discloses a process which is simple, rapid, cost effective, time-saving and industrially convenient process.

### Background of the Invention

Lisdexamfetamine dimesylate is used to treat attention deficit hyperactivity disorder (ADHD) in adults and children 6 years of age and older. This medicine is also used to treat moderate to severe binge eating disorder (BED). It belongs to the group of medicines called central nervous system (CNS) stimulants.

Lisdexamfetamine dimesylate increases attention and decreases restlessness in children and adults who are overactive, cannot concentrate for very long, or are easily distracted and impulsive. This medicine is used as part of a total treatment program that also includes social, educational, and psychological treatment.

Lisdexamfetamine dimesylate also referred to as I-lysine-d-amphetamine dimesylate of Formula I is chemically named as (2S)-2,6-Diamino-N-[(1S)-1-methyl-2-phenylethyl]hexanamide dimesylate.

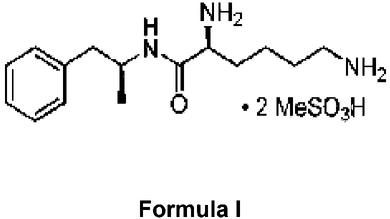

Lisdexamfetamine dimesylate is a white to off-white powder, very soluble in water and soluble in methanol, melting point 192-198°C, molecular formula C₁₇H₃₃N₃O₇S₂ and molecular weight 455.59 g/mol.

Lisdexamfetamine is currently commercially available only as 10, 20, 30, 40, 50 and 60 mg hard capsules or chewable tablets. Each capsule or chewable tablet contains 10, 20, 30, 40, 50 or 60 mg lisdexamfetamine dimesylate, equivalent to 5.8 mg, 11.6 mg, 17.3 mg, 23.1 mg, 28.9 mg or 34.7 mg lisdexamfetamine, respectively.

Effervescent tablets are designed to release carbon dioxide with rapid reaction with water and disintegrate rapidly when dropped in liquid. They have gained considerable attention as a preferred alternative to conventional tablets and capsules due to their better patient compliance. Today, there are a growing number of people who cannot swallow tablets or capsules. Effervescent dosage forms is ideal for deliquescent or hygroscopic active substances. Both effervescent dosage form manufacturing and moisture absorbing materials require special attention and humidity controlled environment during manufacturing processes. On the other side, in effervescent tablet formulations the active ingredient is already dissolved before its administration and effervescent dosage form is easier to take compared to tablets or capsules.

Swallowing big tablets or capsules is difficult for the patients. Effervescent technology provides an alternative to them. Disintegration and dissolution of standard tablets also takes additional time in the stomach. In effervescent dosage form, ingredients are distributed or dissolved in the solution, that enables rapid absorption and overcomes swallowing difficulties. They can be taken in liquids and promotes patients to take more liquid.

The patent WO2006121552A2 discloses a capsule and chewable tablet formulation containing lisdexamfetamine dimesylate, microcrystalline cellulose, croscarmellose sodium, magnesium stearate.

In the prior art have described the capsule and chewable tablet formulations of lisdexamfetamine. The possibility of an effervescent tablet form has been mentioned, but no example formulation has been provided.

Lisdexamfetamine dimesylate has some disadvantages in formulation due to its prodrug structure, stability problems, difficulties in patient compliance and dose adjustments. Since lisdexamfetamine dimesylate is a deliquescent active substance, maintaining stability is quite challenging. The use of lisdexamfetamine dimesylate in effervescent tablet form has become advantageous in terms of patient compliance as well as improving stability by reducing the interaction of the drug with moisture in manufacturing and packaging. Therefore, a formulation in effervescent tablet form with high stability and patient compliance is needed.

### Detailed Description of the Invention

The main object of the present invention provides an effervescent tablet formulation comprising lisdexamfetamine dimesylate having the desired stability and high patient compliance.

Another object of the present invention is to obtain an effervescent tablet formulation comprising lisdexamfetamine dimesylate having flowability.

Another object of the present invention is to provide a preparation process of the effervescent tablet formulation comprising lisdexamfetamine dimesylate which is a simple and cost-effective process.

Lisdexamfetamine dimesylate is a deliquescent active substance. The deliquescent properties of lisdexamfetamine dimesylate may cause liquefaction in moist environment, which may affect the potency and stability of the drug. It is an advantage this active ingredient to manufacture and store the product in tablet form with low moisture content and use it by dissolving in water. This improves stability by limiting moisture absorption of the active substance in finished dosage form of moisture with the drug. Therefore, our study of the formulation of lisdexamfetamine dimesylate in effervescent tablet form allowed us to obtain a formulation with high stability and high patient compliance.

According to one embodiment of the invention, an effervescent tablet formulation comprising lisdexamfetamine dimesylate and at least one pharmaceutically acceptable excipient.

According to one embodiment of the present invention, the amount of the lisdexamfetamine dimesylate is between 0.1% and 10.0% by weight of the total formulation.

According to one embodiment, pharmaceutically acceptable excipient is selected from the group comprising acid sources, gas generating agents, fillers, lubricants, sweeteners, flavouring agents, stabilizers, disintegrants, coloring agents or mixtures thereof.

Suitable acid sources are selected from the group comprising citric acid, malic acid, nicotinic acid, ascorbic acid, acetylsalicylic acid, lactic acid, maleic acid, acid, adipic acid, tartaric acid or mixtures thereof.

According to this embodiment of the present invention, the amount of acid source is between 5.0% and 35.0% by weight in the total formulation. Preferably, the amount of acid source is between 10.0% and 30.0% by weight in the total formulation.

According to this embodiment of the present invention, the acid source is citric acid or malic acid or mixture thereof.

We chose citric acid and malic acid as acid sources. These two acids, when combined with sweeteners and other compounds, can have synergic effects on the flavour profile and mask some undesirable taste. In this way, less sweeteners are used.

Suitable gas generating agents are selected from the group comprising sodium bicarbonate, sodium sesquicarbonate, potassium carbonate, calcium carbonate sodium carbonate anhydrous, calcium carbonate, potassium bicarbonate, sodium carbonate, sodium glycine carbonate or mixtures thereof.

According to this embodiment of the present invention, the amount of gas generating agent is between 10.0% and 35.0% by weight in the total formulation. Preferably, the amount of gas generating agent is between 15.0% and 30.0% by weight in the total formulation.

According to an embodiment of the present invention, the gas generating agent is sodium bicarbonate.

Gas generating in effervescent tablet formulations is results from neutralization reaction of acid and base of acid and base substances such as carbonates or bicarbonates as base and citric acid or malic acid as acid that react rapidly in the presence of water and release CO₂ and provide a carbonated or sparkling liquid drink. Presence of neutralization reaction in effervescent dosage forms gives an advantage of better taste masking compared to other oral dosage forms.

Suitable fillers are selected from the group comprising microcrystalline cellulose, mannitol , D-mannitol, maltodextrin, lactose, starch, sucrose, ammonium alginate, calcium phosphate, dibasic calcium phosphate, neutral pellets, calcium sulfate, cellulose acetate, erythritol, ethyl cellulose, fructose, glyceryl palmitostearate, lactose, magnesium carbonate, magnesium oxide, maltose, medium chain triglycerides, polydextrose, polymethacrylates, sodium chloride, sorbitol, sugar spheres, tragacanth, trehalose, polysorbate 80 or mixtures thereof.

According to this embodiment of the present invention, the filler is microcrystalline cellulose or mannitol or D-mannitol or maltodextrin or mixture thereof.

According to this embodiment of the present invention, the amount of filler is between 20.0% and 60.0% by weight of the total formulation, preferably it is between 25.0% and 55.0% by weight of the total formulation.

Suitable lubricants are selected from the group comprising sodium stearyl fumarate, magnesium stearate, talc, colloidal silicon dioxide, calcium stearate, zinc stearate, magnesium lauryl sulfate, sodium oleate, sodium acetate, stearic acid, fumaric acid, or mixtures thereof.

According to one embodiment of the present invention, the lubricant is sodium stearyl fumarate or magnesium stearate or mixture thereof.

According to one embodiment of the present invention, the amount of lubricant is between 0.01% and 5.0% by weight in the total formulation. This ratio provides good flowability in the formulation.

Suitable sweeteners are selected from the group comprising, aspartame, xylitol, sucralose, sorbitol, erythritol, thaumatin, mogroside, inulin, acesulfame-K, saccharin or its sodium and calcium salts, sodium cyclamate, sucrose, glucose, menthol, peppermint, cinnamon or mixtures thereof.

According to this embodiment of the present invention, the amount of sweetener is between 0.1% and 20.0% by weight in the total formulation. Preferably, the amount of sweetener is between 1.0% and 15.0% by weight in the total formulation.

According to an embodiment of the present invention, the sweeteners is aspartam or xylitol or mixtures thereof.

Suitable flavouring agents are selected from the group comprising orange flavour, cherry flavour, lemon flavour, menthol, peppermint, cinnamon, chocolate, vanillin, strawberry, grape, black currant, raspberry, banana, red fruits, wild berries or mixtures thereof.

According to this embodiment of the present invention, the amount of flavouring agent is between 0.001% and 10.0% by weight in the total formulation.

According to an embodiment of the present invention, the flavouring agent is orange flavour or lemon flavour or mixtures thereof.

Suitable stabilizers are selected from the group comprising magnesium aluminometasilicate, glycerol dibehenate, citric acid, fumaric acid, tartaric acid, sodium citrate, sodium benzoate, sodium dihydrogen phosphate, calcium carbonate, magnesium carbonate, arginine, lysine, meglumine, ascorbic acid, gallic acid esters or the mixtures thereof.

According to an embodiment of the present invention, the stabilizer is magnesium aluminometasilicate. When these were used, thus obtaining a formulation with high stability.

According to one embodiment of the present invention, the amount of the stabilizer is between 1.0% and 20.0% by weight of the total formulation. Preferably, it is between 5.0% and 15.0% by weight in the total formulation.

Suitable disintegrants are selected from the group comprising crospovidone, croscarmellose sodium, sodium starch glycolate, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

According to one embodiment of the present invention, the disintegrant is crospovidone.

According to one embodiment of the present invention, the amount of disintegrant is between 0.01% and 10.0% by weight in the total formulation.

Suitable coloring agents are selected from the group comprising beta-carotene (E160a), ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&Cblue, carmoisine indigotine (indigo Carmine); iron oxides (such as; iron oxide red, yellow, black), quinoline yellow, flaming red, carmine, carmoisine, sunset yellow or mixtures thereof.

According to one embodiment of the present invention, the coloring agents is beta-carotene.

According to one embodiment of the present invention, the amount of coloring agents is between 0.01% and 5.0% by weight in the total formulation.

### Example 1;

| **Ingredients** | **%by weight** | **%by weight** |
|---|---|---|
| Lisdexamfetamine Dimesylate | 2.9 | 0.1-10 |
| Mannitol | 42.5 | 30-55 |
| Sodium Bicarbonate | 21.4 | 10-30 |
| Citric acid | 18.6 | 5-25 |
| Aspartam | 2.3 | 0.1-10 |
| Maltodextrin | 8.6 | 1-15 |
| Sodium Stearyl Fumarate | 0.6 | 0.01-5 |
| Orange flavour | 2.3 | 0.1-10 |
| Beta-carotene (E160a) | 0.8 | 0.01-5 |
| **TOTAL** | **100** | **100** |

a) sieving all components through 0.8 mm sieve and geometric mixing with blender,
b) compressing the resulting mixture with an appropriate punch.

### Example 2;

| **Ingredients** | **%by weight** | **%by weight** |
|---|---|---|
| Lisdexamfetamine Dimesylate | 2.9 | 0.1-10 |
| Microcrystalline Cellulose | 29.4 | 20-40 |
| Sodium Bicarbonate | 23.1 | 15-35 |
| Malic Acid | 24.6 | 15-35 |
| Xylitol | 10 | 1-20 |
| Magnesium Aluminometasilicate | 9.1 | 1-20 |
| Sodium Stearyl Fumarate | 0.9 | 0.01-5 |
| **TOTAL** | **100** | **100** |

a) sieving all components through 0.8 mm sieve and geometric mixing with blender,
b) compressing the resulting mixture with an appropriate punch.

### Example 3;

| **Ingredients** | **%by weight** | **%by weight** |
|---|---|---|
| Lisdexamfetamine Dimesylate | 2.9 | 0.01-10 |
| Sodium Bicarbonate | 21.4 | 10-30 |
| Citric acid | 18.6 | 10-30 |
| D-mannitol | 14.7 | 5-25 |
| Xylitol | 8.6 | 1-15 |
| Microcrystalline Cellulose | 22.8 | 10-30 |
| Crospovidone | 2.3 | 0.01-10 |
| Magnesium Aluminometasilicate | 8.6 | 1-15 |
| Magnesium stearate | 0.1 | 0.01-5 |
| Lemon flavour | 0.01 | 0.001-1 |
| **TOTAL** | **100** | **100** |

a) sieving all components through 0.8 mm sieve and geometric mixing with blender,
b) compressing the resulting mixture with an appropriate punch.

## Claims

1. An effervescent tablet formulation comprising lisdexamfetamine dimesylate and at least one excipient.

2. The effervescent tablet formulation according to claim 1, wherein the amount of the lisdexamfetamine dimesylate is between 0.1% and 10.0% by weight of the total formulation.

3. The effervescent tablet formulation according to claim 1, wherein excipient is selected from the group comprising acid sources, gas generating agents, fillers, lubricants, sweeteners, flavouring agents, stabilizers, disintegrants, coloring agents or mixtures thereof.

4. The effervescent tablet formulation according to claim 3, wherein acid sources are selected from the group comprising citric acid, malic acid, nicotinic acid, ascorbic acid, acetylsalicylic acid, lactic acid, maleic acid, acid, adipic acid, tartaric acid or mixtures thereof.

5. The effervescent tablet formulation according to claim 3 or 4, wherein the acid source is citric acid or malic acid or mixture thereof.

6. The effervescent tablet formulation according to claim 3, wherein gas generating agents are selected from the group comprising sodium bicarbonate, sodium sesquicarbonate, potassium carbonate, calcium carbonate sodium carbonate anhydrous, calcium carbonate, potassium bicarbonate, sodium carbonate, sodium glycine carbonate or mixtures thereof.

7. The effervescent tablet formulation according to claim 3 or 6, wherein the gas generating agent is sodium bicarbonate.

8. The effervescent tablet formulation according to claim 3, wherein fillers are selected from the group comprising microcrystalline cellulose, mannitol, D-mannitol, maltodextrin, lactose, starch, sucrose, ammonium alginate, calcium phosphate, dibasic calcium phosphate, neutral pellets, calcium sulfate, cellulose acetate, erythritol, ethyl cellulose, fructose, glyceryl palmitostearate, lactose, magnesium carbonate, magnesium oxide, maltose, medium chain triglycerides, polydextrose, polymethacrylates, sodium chloride, sorbitol, sugar spheres, tragacanth, trehalose, polysorbate 80 or mixtures thereof.

9. The effervescent tablet formulation according to claim 3 or 8, wherein the filler is microcrystalline cellulose or mannitol or D-mannitol or maltodextrin or mixture thereof.

10. The effervescent tablet formulation according to claim 3, wherein lubricants are selected from the group comprising sodium stearyl fumarate, magnesium stearate, talc, colloidal silicon dioxide, calcium stearate, zinc stearate, magnesium lauryl sulfate, sodium oleate, sodium acetate, stearic acid, fumaric acid, or mixtures thereof.

11. The effervescent tablet formulation according to claim 3 or 10, wherein the lubricant is sodium stearyl fumarate or magnesium stearate or mixture thereof.

12. The effervescent tablet formulation according to claim 3, wherein sweeteners are selected from the group comprising, aspartame, xylitol, sucralose, sorbitol, erythritol, thaumatin, mogroside, inulin, acesulfame-K, saccharin or its sodium and calcium salts, sodium cyclamate, sucrose, glucose, menthol, peppermint, cinnamon or mixtures thereof.

13. The effervescent tablet formulation according to claim 3 or 12, wherein the sweeteners is aspartam or xylitol or mixtures thereof.

14. The effervescent tablet formulation according to claim 3, wherein stabilizers are selected from the group comprising magnesium aluminometasilicate, glycerol dibehenate, citric acid, fumaric acid, tartaric acid, sodium citrate, sodium benzoate, sodium dihydrogen phosphate, calcium carbonate, magnesium carbonate, arginine, lysine, meglumine, ascorbic acid, gallic acid esters or the mixtures thereof.

15. The effervescent tablet formulation according to claim 3 or 14, wherein the stabilizer is magnesium aluminometasilicate.
